(19) Europäisches Patentamt
European Patent Office
Office européen des brevets

(11) Veröffentlichungsnummer: **0 189 044 A2**

(12) **EUROPÄISCHE PATENTANMELDUNG**

(21) Anmeldenummer: **86100095.8**

(22) Anmeldetag: **07.01.86**

(51) Int. Cl.[4]: **C 07 D 249/08**, A 01 N 43/653 // C07C49/84

(30) Priorität: **17.01.85 DE 3501370**

(43) Veröffentlichungstag der Anmeldung: **30.07.86 Patentblatt 86/31**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Reinecke, Paul, Dr., Steinstrasse 8, D-5090 Leverkusen 3 (DE)**
Erfinder: **Brandes, Wilhelm, Dr., Eichendorffstrasse 3, D-5653 Leichlingen 1 (DE)**
Erfinder: **Büchel, Karl Heinz, Prof. Dr., Dabringhausener Strasse 42, D-5093 Burscheid (DE)**

(54) **1-Aryl-2,2-dialkyl-2-2(1,2,4-triazol-1-yl)-ethanole.**

(57) Die Erfindung betrifft neue 1-Aryl-2,2-dialkyl-2-(1,2,4-triazol-1-yl)-ethanole, ein Verfahren zu ihrer Herstellung und ihre Verwendung als Fungizide.

Die Verbindungen der allgemeinen Formel (I)

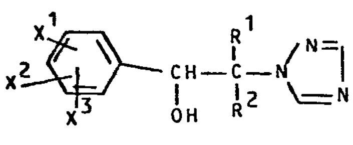

(I)

in welcher $X^1$, $X^2$, $X^3$, $R^1$ und $R^2$ die in der Beschreibung angegebene Bedeutung besitzen, werden erhalten, wenn man die entsprechenden Ethanon-Derivate in bekannter Weise reduziert.

Die erfindungsgemässen Verbindungen sind als Pflanzenschutzmittel insbesondere zur Bekämpfung von Pflanzenkrankheiten in Getreide-, Obst- und Gemüsekulturen von Interesse.

EP 0 189 044 A2

BAYER AKTIENGESELLSCHAFT 5090 Leverkusen, Bayerwerk
Konzernverwaltung RP
Patentabteilung Slr/Kü-c
Ia

# 1-Aryl-2,2-dialkyl-2-(1,2,4-triazol-1-yl)-ethanole

Die Erfindung betrifft neue 1-Aryl-2,2-dialkyl-2-(1,2,4-triazol-1-yl)-ethanole, ein Verfahren zu ihrer Herstellung und ihre Verwendung als Fungizide.

Es ist bereits bekannt, daß bestimmte 1-Aryl-2,2-dialkylazolyl-ethanone wie beispielsweise das 1-(4-Fluorphenyl)-2,2-dimethyl-2,(1,2,4-triazol-1-yl)-ethanon, das 1-/4-(4-Chlorphenoxy)-phenyl7-2,2-dimethyl-2-imidazol-1-ylethanon oder das 1-/4-(4-Chlorphenyl)-phenyl7-2,2-dimethyl-2-imidazol-1-yl-ethanon eine gute antimykotische Wirksamkeit besitzen, und auch als Pflanzenschutzmittel eingesetzt werden können (vgl. z.B. DE-OS 3 307 218).

Die Wirkung dieser vorbekannten Verbindungen ist jedoch insbesondere bei niedrigen Aufwandmengen und -konzentrationen nicht immer in allen Anwendungsbereichen völlig zufriedenstellend.

Es wurden neue 1-Aryl-2,2-dialkyl-2-(1,2,4-triazol-1-yl)-ethanole der allgemeinen Formel (I),

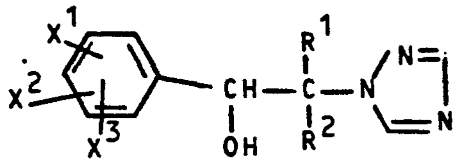

(I)

in welcher

$X^1$, $X^2$ und $X^3$ jeweils unabhängig voneinander für Halogen, Alkyl, Alkoxy, Alkylthio, Halogenalkyl, Halogenalkoxy, Halogenalkylthio, Nitro, Cyano, Hydroxycarbonyl, Alkoxycarbonyl, Hydroximinoalkyl, Alkoximinoalkyl sowie jeweils gegebenenfalls substituiertes Phenyl, Phenoxy, Benzyl oder Benzyloxy stehen,

wobei

$X^2$ und/oder $X^3$ auch für Wasserstoff stehen können, wenn gleichzeitig $X^1$ für Alkylthio, Halogenalkoxy, Halogenalkylthio, Nitro, Cyano, Hydroxy, Hydroxycarbonyl, Alkoxycarbonyl, Hydroximinoalkyl, Alkoximinoalkyl sowie für jeweils gegebenenfalls substituiertes Phenoxy, Benzyl oder Benzyloxy oder für substituiertes Phenyl steht,

und

$R^1$ und $R^2$ unabhängig voneinander für Alkyl stehen,

sowie deren pflanzenverträgliche Säureadditionssalze und Metallsalzkomplexe gefunden.

Die Verbindungen der Formel (I) können als optische Isomere und gegebenenfalls auch als Diastereomere vorliegen. Sowohl die einzelnen Isomeren als auch deren Gemische werden erfindungsgemäß beansprucht.

Weiterhin wurde gefunden, daß man die neuen 1-Aryl-2,2-dialkyl-2-(1,2,4-triazol-1-yl)-ethanole der allgemeinen Formel (I), sowie deren pflanzenverträgliche Säureadditionssalze und Metallsalzkomplexe erhält, wenn man 1-Aryl-2,2-dialkyl-2-(1,2,4-triazol-1-yl)-ethanone der Formel (II),

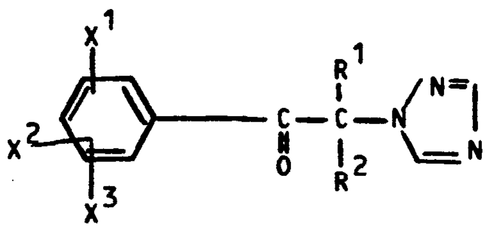

(II)

in welcher

$X^1$, $X^2$, $X^3$, $R^1$ und $R^2$ die oben angegebene Bedeutung haben,

nach bekannten Methoden in üblicher Weise reduziert,

und gegebenenfalls anschließend eine Säure oder ein Metallsalz addiert.

Schließlich wurde gefunden, daß die neuen 1-Aryl-2,2-dialkyl-2-(1,2,4-triazol-1-yl)-ethanole der Formel (I) sowie deren pflanzenverträgliche Säureadditions-Salze bzw. Metallsalz-Komplexe fungizide Eigenschaften besitzen.

Überraschenderweise zeigen die erfindungsgemäßen 1-Aryl-2,2-dialkyl-2-(1,2,4-triazol-1-yl)-ethanole der Formel (I) eine erheblich bessere fungizide Wirksamkeit als die aus dem Stand der Technik bekannten 1-Aryl-2,2-dialkyl-2-azolylethanone, wie beispielsweise das 1-(4-Fluorphenyl)-2,2-dimethyl-2-(1,2,4-triazol-1-yl)-ethanon, das 1-[4-(4-Chlorphenoxy)-phenyl]-2,2-dimethyl-2-imidazol-1-ylethanon oder das 1-[4-(4-Chlorphenyl)-phenyl]-2,2-dimethyl-2-imidazol-1-ylethanon, welches chemisch und wirkungsmäßig naheliegende Verbindungen sind.

Die erfindungsgemäßen 1-Aryl-2,2-dialkyl-2-(1,2,4-triazol-1-yl)-ethanole der Formel (I) stellen somit einen Fortschritt im Stand der Technik dar.

Die neuen 1-Aryl-2,2-dialkyl-2-(1,2,4-triazol-1-yl)-ethanole sind durch die Formel (I) allgemein definiert. Bevorzugt sind die neuen Verbindungen der Formel (I), bei welchen

$X^1$, $X^2$ und $X^3$ jeweils unabhängig voneinander für Halogen, Alkyl mit 1 bis 4 Kohlenstoffatomen, Alkoxy oder Alkylthio mit jeweils 1 bis 4 Kohlenstoffatomen, Halogenalkyl, Halogenalkoxy oder Halogenalkylthio mit jeweils 1 bis 2 Kohlenstoff- und

1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy stehen,

wobei $X^2$ und/oder $X^3$ auch für Wasserstoff stehen können, wenn gleichzeitig $X^1$ für Alkylthio mit 1 bis 4 Kohlenstoffatomen, Halogenalkoxy oder Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und/oder Alkyl mit 1 bis 4 Kohlenstoffatomen substituiertes Phenoxy, Benzyl oder Benzyloxy oder für durch Halogen und/oder Alkyl mit 1 bis 2 Kohlenstoffatomen substituiertes Phenyl steht,

und

$R^1$ und $R^2$ jeweils unabhängig voneinander für geradkettiges oder verzweigtes Alkyl mit 1 bis 6 Kohlenstoffatomen stehen.

Besonders bevorzugt sind diejenigen Verbindungen der Formel (I), in denen

$X^1$, $X^2$ und $X^3$ jeweils unabhängig voneinander für Fluor, Chlor, Brom, Methyl, Ethyl, Isopropyl, tert.-Butyl, Methoxy, Methylthio, Nitro, Cyano, Hydroxy, Hydroxycarbonyl, Ethoxycarbonyl, Hydroximinomethyl, 1-Hydroximinoethyl, Methoxyiminomethyl, 1-Methoximinoethyl, sowie jeweils gegebenenfalls durch Fluor, Chlor, Methyl ein- bis dreifach substituiertes Phenyl, Phenoxy, Benzyl und Benzyloxy stehen,

wobei $X^2$ und/oder $X^3$ auch für Wasserstoff stehen können, wenn gleichzeitig $X^1$ für Methylthio, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl sowie jeweils gegebenenfalls durch Fluor, Chlor und/oder Methyl ein- bis dreifach substituiertes Phenoxy, Benzyl oder Benzyloxy oder für durch Fluor, Chlor und/oder Methyl substituiertes Phenyl steht,

und

$R^1$ und $R^2$ jeweils unabhängig voneinander für geradkettiges oder verzweigtes Alkyl mit 1 bis 4 Kohlenstoffatomen stehen.

Bevorzugte erfindungsgemäße Verbindungen sind auch Additionsprodukte aus Säuren und denjenigen 1-Aryl-2,2-dialkyl -2-(1,2,4-triazin-1-yl)-ethanolen der Formel (I), in denen die Substituenten $X^1$, $X^2$, $X^3$, $R^1$ und $R^2$ die Bedeutungen haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono-, bi-, und trifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie o-Toluolsulfonsäure und 1,5-Naphthalindisulfonsäure.

Im einzelnen seien außer den bei den Herstellungsbeispielen genannten Verbindungen die folgenden 1-Aryl-2,2-dialkyl -2-(1,2,4-triazol-1-yl)-ethanole der allgemeinen Formel (I) genannt:

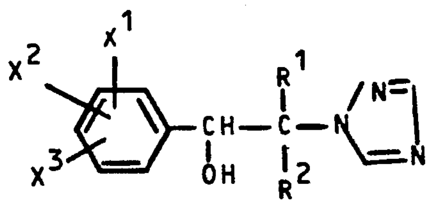 (I)

| 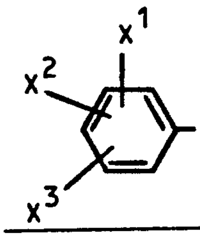 | $R^1$ | $R^2$ |
|---|---|---|
| 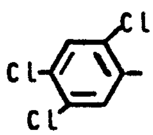 | $CH_3$ | $CH_3$ |
| 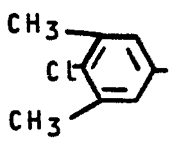 | $CH_3$ | $CH_3$ |
| 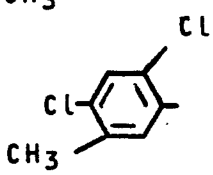 | $CH_3$ | $CH_3$ |
| 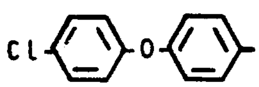 | $CH_3$ | $C_2H_5$ |
| 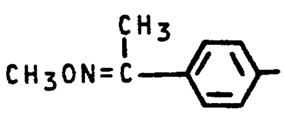 | $CH_3$ | $CH_3$ |

| 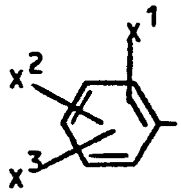 | $R^1$ | $R^2$ |
|---|---|---|
| 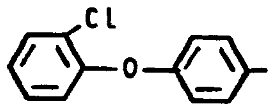 | $CH_3$ | $CH_3$ |
| 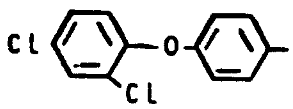 | $CH_3$ | $CH_3$ |
| 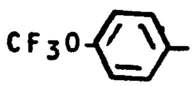 | $CH_3$ | $CH_3$ |
| 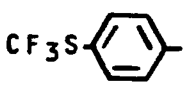 | $CH_3$ | $CH_3$ |
| 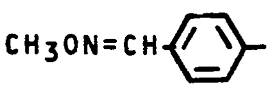 | $CH_3$ | $CH_3$ |
| 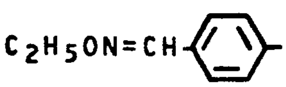 | $CH_3$ | $CH_3$ |

Verwendet man beispielsweise 1-(2,4,6-Trichlorphenyl)-2,2-dimethyl-2-(1,2,4-triazol-1-yl)-ethanol als Ausgangsstoff und Natriumborhydrid als Reduktionsmittel, so läßt sich der Reaktionsablauf des erfindungsgemäßen Verfahrens durch das folgende Formelschema darstellen:

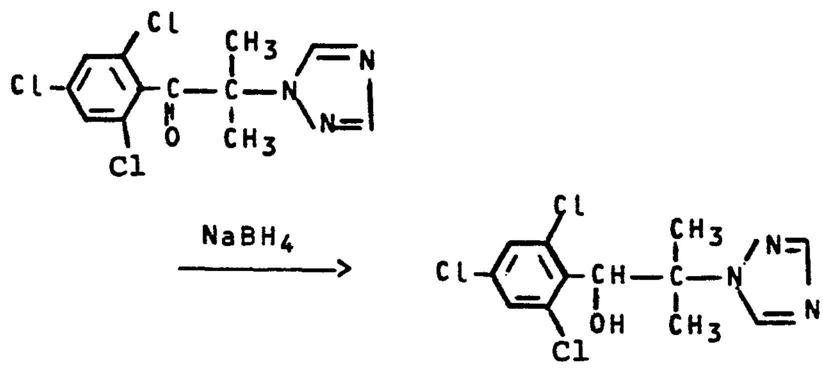


Die zur Durchführung des erfindungsgemäßen Verfahrens als Ausgangsstoffe benötigten 1-Aryl-2,2-dialkyl-2-(1,2,4-triazol-1-yl)-ethanone sind durch die Formel (II) allgemein definiert. In dieser Formel (II) stehen $X^1$, $X^2$, $X^3$, $R^1$ und $R^2$ vorzugsweise für diejenigen Substituenten, die bereits im Zusammenhang mit der Beschreibung der erfindungsgemäßen Stoffe der Formel (I) als bevorzugt für diese Reste genannt wurden.

Die 1-Aryl-2,2-dialkyl-2-(1,2,4-triazol-1-yl)-ethanone der Formel (II) sind größtenteils bekannt (vgl. z.B. DE-OS 3 307 218; DE-OS 3 222 191).

Man erhält sie beispielsweise, wenn man Halogenketone der Formel (III),

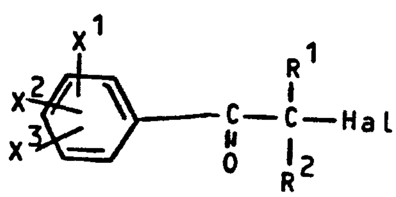

(III)

in welcher

$X^1$, $X^2$, $X^3$, $R^1$ und $R^2$ die oben angegebene Bedeutung haben

und

Hal für Halogen, insbesondere für Chlor oder Brom steht

mit Triazol der Formel (IV),

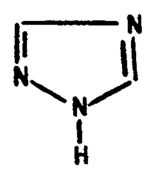

(IV)

gegebenenfalls in Gegenwart eines Verdünnungsmittels, wie beispielsweise Aceton und gegebenenfalls in Gegenwart eines Säurebindemittels, wie beispielsweise Kaliumcarbonat bei Temperaturen zwischen 0° und +120°C umsetzt.

Die Halogenketone der Formel (III) sind bekannt (vgl. z.B. Synth. Commun. 12. 261-266 [1982]) oder können nach bekannten Verfahren erhalten werden, indem man beispielsweise die allgemein bekannten Ketone der Formel (V),

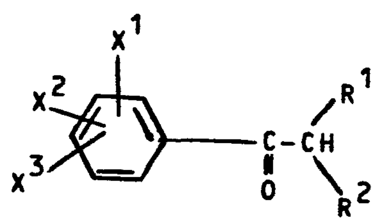

(V)

in welcher

$X^1$,$X^2$, $X^3$, $R^1$ und $R^2$ die oben angegebene Bedeutung haben,

mit Chlor oder Brom gegebenenfalls in Gegenwart eines Verdünnungsmittels, wie beispielsweise Chloroform, mit üblichen Halogenierungsmitteln, wie beispielsweise Sulfurylchlorid bei Temperaturen zwischen 0° und 60°C umsetzt.

Das Triazol der Formel (IV) und die Ketone der Formel (V) sind allgemein bekannte Verbindungen der organischen Chemie.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (II) etwa 0,3 bis 1 Mol eines komplexen Hydrids, wie Natriumborhydrid oder Lithiumalanat, ein. Zur Isolielierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Art und Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (II) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I ) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren,

wie z.B. Essigsäure, Maleinsäure, Bernsteinsäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppen infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise z.B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt.

Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;

Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Drechslera-Arten, wie beispielsweise Drechslera graminea (synonym mit Helminthosporium);
Fusarium-Arten, wie beispielsweise Fusarium culmorum;
Ustilago-Arten, wie beispielsweise Ustilago nuda oder Ustilago avenae;
Septoria-Arten, wie beispielsweise Septoria nodorum;
Tilletia-Arten, wie beispielsweise Tilletia caries;
Xanthomonas-Arten, wie beispielsweise Xanthomonas oryzae;
Pseudomonas-Arten, wie beispielsweise Pseudomonas lachrymans;
Pyricularia-Arten, wie beispielsweise Pyricularia oryzae;
Pellicularia-Arten, wie beispielsweise Pellicularia sasakii, und
Pyrenophora-Arten, wie beispielsweise Pyrenophora teres (Konidienform: Drechslera, Synonym: Helminthosporium);
Leptosphaeria-Arten, wie beispielsweise Leptosphaeria nodorum;
Cochliobolus-Arten, wie beispielsweise Cochliobolus sativus, (Konidienform: Drechslera, Synonym: Helminthosporium);
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Getreidestengelgrundfäule (Fusarium culmorum) außerdem gegen die Erreger Cochliobolus sativus, Pyrenophora teres sowie Drechslera graminea oder zur Bekämpfung von Krankheiten im Obst- und Gemüsebau, wie beispielsweise gegen den Erreger des echten Gurkenmehltaus (Sphaerotheca fuliginea) oder gegen den Erreger des Apfelschorfs (Venturia inaequalis) oder auch zur Bekämpfung von Reiskrankheiten, wie beispielsweise gegen den Erreger der Reisfleckenkrankheit (Pyricularia oryzae) einsetzen. Auch gegen Rostpilze und Cercospora-Arten zeigen die erfindungsgemäßen Wirkstoffe eine gute Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen

in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Beispiel 1

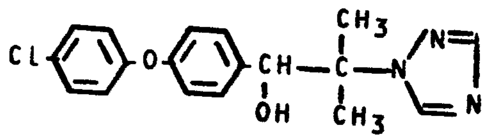

Zu 10 g (0,029 Mol) 1-[4-(4-Chlorphenoxy)-phenyl]-2-methyl-2-(1,2,4,-triazol-1-yl)-propan-1-on in 150 ml Methanol gibt man unter Rühren bei Raumtemperatur 0,3 g (0,008 Mol) Natriumborhydrid in 5 ml Wasser gelöst, rührt eine Stunde nach, engt im Vakuum ein, nimmt den Rückstand in Dichlormethan auf, wäscht mit Wasser, trocknet die Dichlormethanphase über Natriumsulfat und entfernt das Lösungsmittel im Vakuum.

Man erhält 9,5 g (95 % der Theorie) an 1-[4-(4-Chlorphenoxy)-phenyl]-2-methyl-2-(1,2,4-triazol-1-yl)-propan-1-ol vom Schmelzpunkt 134°C.

Herstellung der Ausgangsverbindung:

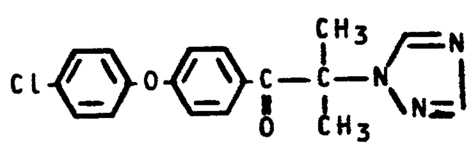

Zu 69 g (1 Mol) 1,2,4-Triazol und 207 g (1,5 Mol) Kaliumcarbonat im 1000 ml Aceton gibt man unter Rühren 180 g (0,51 Mol) 2-Brom-1-/4-(4-chlorphenoxy)-phenyl7-2-methylpropan-1-on und erhitzt anschließend für 10 Stunden auf Rückflußtemperatur. Zur Aufarbeitung läßt man den Reaktionsansatz abkühlen, filtriert, engt das Filtrat im Vakuum ein, nimmt den Rückstand in Dichlormethan auf, wäscht mit Wasser, trocknet über Natriumsulfat und entfernt das Lösungsmittel im Vakuum. Der Rückstand läßt sich aus Diisopropylether umkristallisieren.

Man erhält 64 g (36,7 % der Theorie) an 1-/4-(4-Chlorphenoxy)-phenyl7-2-methyl-2-(1,2,4-triazol-1-yl)-propan-1-on vom Schmelzpunkt 112°C.

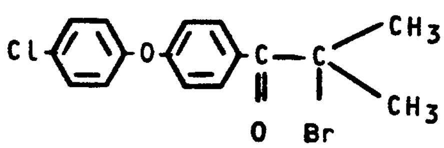

Zu 140 g (0,51 Mol) /4-(4-Chlorphenoxy)-phenyl7-isopropylketon im 500 ml Chloroform gibt man tropfenweise unter Rühren 26 ml (81,64 g bzw. 0,5 Mol) Brom, rührt nach beendeter Zugabe eine Stunde nach und entfernt die flüchtigen Bestandteile der Reaktionsmischung im Vakuum.

Man erhält 180 g (99,5 % der Theorie) an 2-Brom-2-/4-(4-chlorphenoxy)-phenyl7-2-methylpropan-1-on, welches ohne weitere Reinigung in die nächste Reaktionsstufe eingesetzt werden kann.

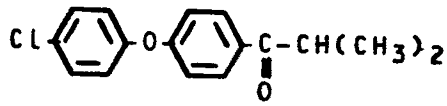

Zu 285 g (1,4 Mol) 4-Chlordiphenylether im 1000 ml 1,2-Dichlorethan gibt man bei Raumtemperatur (gegebenenfalls Kühlung) unter Rühren zunächst 226,6 g (1,7 Mol) Aluminium-III-chlorid und dann 160 g (1,5 Mol) Isobuttersäurechlorid. Nach beendeter Zugabe rührt man 3 Stunden nach, gießt den Reaktionsansatz auf 2 l Eiswasser, wäscht die organische Phase mit Wasser, trocknet über Natriumsulfat und entfernt das Lösungsmittel im Vakuum. Der Rückstand wird im Hochvakuum destilliert.

Man erhält 290 g (75,7 % der Theorie) an /4-(4-Chlorphenoxy)-phenyl7-isopropyl-keton vom Siedepunkt 160°C bei 0,2 mbar.

In entsprechender Weise und gemäß den allgemeinen Herstellungsangaben erhält man die folgenden Verbindungen der allgemeinen Formel (I):

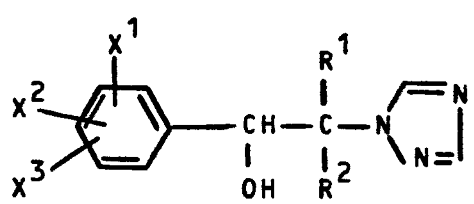

(I)

| Bsp.Nr. | $X^1$ | $X^2$ | $X^3$ | $R^1$ | $R^2$ | Schmelzpunkt (°C) |
|---|---|---|---|---|---|---|
| 2 | 4-Cl-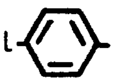 | H | H | $CH_3$ | $CH_3$ | 202 |
| 3 | 2-Cl | 4-Cl | 5-F | $CH_3$ | $CH_3$ | 190 |
| 4 | 4-$CF_3O$ | H | H | $CH_3$ | $CH_3$ | 90 |
| 5 | 4-$CF_3S$ | H | H | $CH_3$ | $CH_3$ | 104 |

Anwendungsbeispiele:

In den folgenden Anwendungsbeispielen werden die nachstehend aufgeführten Verbindungen als Vergleichssubstanzen eingesetzt:

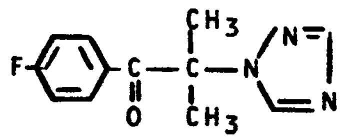

(A)

1-(4-Fluorphenyl)-2,2-dimethyl-2-(1,2,4-triazol-1-yl)-ethanon

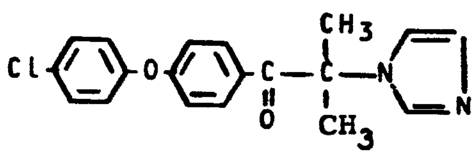

(B)

1-[4-(4-Chlorphenoxy)-phenyl]-2,2-dimethyl-2-(imidazol-1-yl-)ethanon

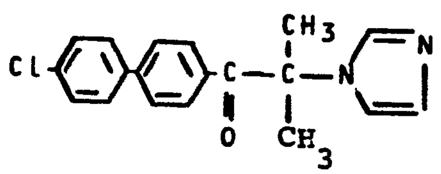

(C)

1-[4-(4-Chlorphenyl)-phenyl]-2,2-dimethyl-2-(imidazol)-1-yl)-ethanon

(alle bekannt aus DE-OS 3 307 218).

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel: 1

Beispiel B

Venturia-Test (Apfel) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel: 1

Patentansprüche

1. 1-Aryl-2,2-dialkyl-2-(1,2,4-triazol-1-yl)-ethanole der allgemeinen Formel (I),

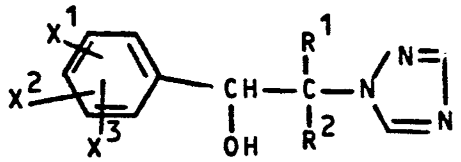

(I)

in welcher

$X^1$, $X^2$ und $X^3$ jeweils unabhängig voneinander für Halogen, Alkyl, Alkoxy, Alkylthio, Halogenalkyl, Halogenalkoxy, Halogenalkylthio, Nitro, Cyano, Hydroxycarbonyl, Alkoxycarbonyl, Hydroximinoalkyl, Alkoximinoalkyl sowie jeweils gegebenenfalls substituiertes Phenyl, Phenoxy, Benzyl oder Benzyloxy stehen,

wobei

$X^2$ und/oder $X^3$ auch für Wasserstoff stehen können wenn gleichzeitig $X^1$ für Alkylthio, Halogenalkoxy, Halogenalkylthio, Nitro, Cyano, Hydroxy, Hydroxycarbonyl, Alkoxycarbonyl, Hydroximinoalkyl, Alkoximinoalkyl sowie für jeweils gegebenenfalls substituiertes Phenoxy, Benzyl oder Benzyloxy oder für substituiertes Phenyl steht,

und

$R^1$ und $R^2$ unabhängig voneinander für Alkyl stehen,

sowie deren pflanzenverträgliche Säureadditionssalze und Metallsalzkomplexe.

2. Verbindungen der allgemeinen Formel (I) in Anspruch 1,

wobei

$X^1$, $X^2$ und $X^3$ jeweils unabhängig voneinander für Halogen, Alkyl mit 1 bis 4 Kohlenstoffatomen, Alkoxy oder Alkylthio mit jeweils 1 bis 4 Kohlenstoffatomen, Halogenalkyl, Halogenalkoxy oder Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy stehen,

wobei $X^2$ und/oder $X^3$ auch für Wasserstoff stehen können, wenn gleichzeitig $X^1$ für

Alkylthio mit 1 bis 4 Kohlenstoffatomen, Halogenalkoxy oder Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und/oder Alkyl mit 1 bis 4 Kohlenstoffatomen substituiertes Phenoxy, Benzyl oder Benzyloxy oder für durch Halogen und/oder Alkyl mit 1 bis 2 Kohlenstoffatomen substituiertes Phenyl steht,

und

$R^1$ und $R^2$ jeweils unabhängig voneinander für geradkettiges oder verzweigtes Alkyl mit 1 bis 6 Kohlenstoffatomen stehen.

3. Verfahren zur Herstellung von 1-Aryl-2,2-dialkyl-2-(1,2,4-triazol-1-yl)-ethanole der allgemeinen Formel (I),

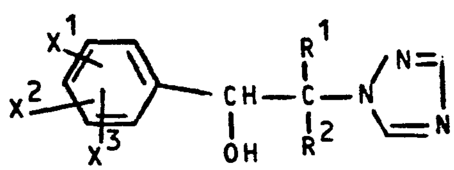

(I)

in welcher

$X^1$, $X^2$ und $X^3$ jeweils unabhängig voneinander für Halogen, Alkyl, Alkoxy, Alkylthio, Halogenalkyl, Halogenalkoxy, Halogenalkylthio, Nitro, Cyano, Hydroxycarbonyl, Alkoxycarbonyl, Hydroximinoalkyl, Alkoximinoalkyl sowie jeweils gegebenenfalls substituiertes Phenyl, Phenoxy, Benzyl oder Benzyloxy stehen,

wobei

$X^2$ und/oder $X^3$ auch für Wasserstoff stehen können wenn gleichzeitig $X^1$ für Alkylthio, Halogenalkoxy, Halogenalkylthio, Nitro, Cyano, Hydroxy, Hydroxycarbonyl, Alkoxycarbonyl, Hydroximinoalkyl, Alkoximinoalkyl sowie für jeweils gegebenenfalls substituiertes Phenoxy, Benzyl oder Benzyloxy oder für substituiertes Phenyl steht,

und

$R^1$ und $R^2$ unabhängig voneinander für Alkyl stehen,

sowie deren pflanzenverträglichen Säureadditionssalzen und Metallsalzkomplexen, dadurch gekennzeichnet, daß man 1-Aryl-2,2-dialkyl-2-(1,2,4-triazol-1-yl)-ethanole der Formel (II),

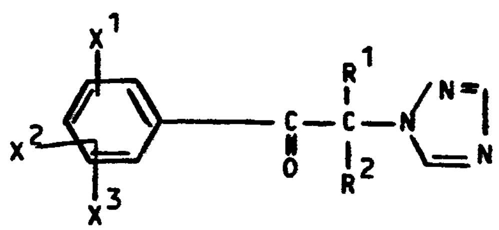

(II)

in welcher

$X^1$, $X^2$, $X^3$, $R^1$ und $R^2$ die oben angegebene Bedeutung haben,

nach bekannten Methoden in üblicher Weise reduziert,

und gegebenenfalls anschließend eine Säure oder ein Metallsalz addiert.

4. Fungizide Mittel, gekennzeichnet durch einen Gehalt an mindestens einem 1-Aryl-2,2-dialkyl-2-(1,2,4-triazol-1-yl)-ethanol der Formel (I). bzw. einem pflanzenverträglichen Säureadditions-Salz oder Metallsalz-Komplex einer Verbindung der Formel (I).

5. Verfahren zur Bekämpfung von Pilzen, dadurch gekennzeichnet, daß man 1-Aryl-2,2-dialkyl-2-(1,2,4-triazol-1-yl)-ethanole der Formel (I) oder deren pflanzenverträgliche Säureadditions-Salze bzw. Metallsalz-Komplexe auf Pilze oder ihren Lebensraum einwirken läßt.

6. Verwendung von 1-Aryl-2,2-dialkyl-2-(1,2,4-triazol-1-yl)-ethanolen der Formel (I) oder von deren pflanzenverträglichen Säureadditions-Salzen bzw. deren Metallsalz-Komplexen als Pflanzenschutzmittel.

7. Verwendung von 1-Aryl-2,2-dialkyl-2-(1,2,4-triazol-1-yl)-ethanolen der Formel (I) oder von deren pflanzenverträglichen Säureadditions-Salzen bzw. deren Metallsalz-Komplexen zur Bekämpfung von Pilzen.

8. **Verfahren zur Herstellung von fungiziden Mitteln, dadurch gekennzeichnet, daß man 1-Aryl-2,2-dialkyl-2-(1,2,4-triazol-1-yl)-ethanole der For**mel (I) bzw. deren pflanzenverträgliche Säureadditions-Salze oder Metallsalz-Komplexe mit Streckmitteln und/oder oberflächen-aktiven Mitteln vermischt.